# EUROPEAN PATENT SPECIFICATION

(11) **EP 0 878 205 B1**
(45) Date of publication and mention of the grant of the patent: **27.09.2006**
(21) Application number: 98250165.2
(22) Date of filing: 14.05.1998
(51) Int. Cl.: A61L 27/00, A61L 31/00

(54) **Implant and process for the production of an implant**
Implantat und Verfahren zur Herstellung eines Implantats
Implant et procédé de production d'un implant

(30) Priority: 16.05.1997 DE 19721876
(43) Date of publication of application: 18.11.1998
(73) Proprietor: Ethicon GmbH, 22851 Norderstedt (DE)
(72) Inventor: Walther, Christoph, Dr., 24568 Kattendorf (DE); Landgrebe, Susanne, 22851 Norderstedt (DE); Hoepffner, Hans-Jochen, Dr., 25335 Bokholt-Hanredder (DE)
(74) Representative: UEXKÜLL & STOLBERG

(56) References cited:
- EP-A- 0 109 197
- EP-A- 0 499 204
- DE-C- 3 830 005
- GB-A- 2 141 435
- US-A- 4 633 873

## Description

The invention relates to an implant which has a an areal or a voluminous form, and a process for the production of such an implant.

An implant with an areal or a voluminous form is to be understood as meaning a three-dimensional implant which extends substantially along a surface or is also extended into the third dimension, in contrast to a thread or to surgical suture material.

Such implants are used, for example, for plugging a bone defect, whereby seeping haemorrhages from the bone are to be stopped effectively. One field of use of such implants is stopping of haemorrhages in oral bone defects, such as occur, for example, after tooth extractions.

Although implants which are already known can provide an adequate mechanical stability and therefore an adequate stabilization of blood clots, they then impede construction of bone through the defect, since they are not degraded or are degraded only very slowly. Conversely, if an agent such as, for example, collagen foam is used for filling the bone defect, although bone growth is not impaired later, reliable stabilization of blood clots cannot be achieved with this.

GB 2 141 435 A discloses structural surgical elements made from a bioabsorbable polymer having a glycolic ester linkage which disintegrate into fragments suitable for removal from or passage through the body without the need for waiting until the material is absorbed. This is achieved by modifiying the elements by means of irradiation or hydrolytic treatment.

DE 38 30 005 C shows an areal implant made from at least two different absorbable filament materials having different melting points. The implant is formed at a temperature which is between these melting points.

EP 0 109 197 A discloses absorbable hemostatic fabric materials of filaments comprising homopolymers or copolymers of lactide and glycolide, preferably polyglactin 910. The fabric may be woven or knitted or formed as a non-woven felt or fabric, and after it has been shaped in this way it is exposed to radiation in excess of 5 Mrads whereby the in vivo absorption time is reduced to less than about 28 days.

The object of the invention is to provide an implant (and a process for the production of such an implant) which on the one hand ensures reliable stopping of haemorrhages in the event of bone defects, and on the other hand does not interfere with construction of bone through the defect in the course of the healing process.

This object is achieved by an implant having the features of claim 8 and by a process having the features of claim 1.

Advantageous versions of the invention can be seen from the subclaims.

The implant according to the invention has an areal or a voluminous form and comprises at least one absorbable yarn which has been predegraded by irradiation and/or chemical treatment. The proportion of the predegraded absorbable yarn is at least 50%, based on the total weight of the implant.

When the absorbable yarn is subjected to treatment for predegradation, its molecular structure is attacked. This leads to the yarn being degraded faster in vivo after an operation. The absorption time, i.e. the period of time after which residues of the implant are no longer detectable in vivo, can be adjusted here by suitable choice of the treatment for the predegradation (irradiation and/or chemical treatment) and of the process parameters used during this (see below). Thus, for example, an absorption time of the absorbable yarn of about 30 days can be chosen. As a result, only very small or even no residues of implant impede the sprouting-in of regenerated bone platelets in the time span of more than 30 days after an operation, which is relevant for bone regeneration.

The absorbable yarn of the implant is merely predegraded and not completely degraded by irradiation and/or chemical treatment. The implant therefore has an adequate mechanical stability, even some days after the surgical intervention. This ensures reliable stopping of haemorrhages, for example in oral bone defects. Since the implant comprises yarn, it is absorbent, so that it can absorb blood without problems.

The pore content in the implant is preferably at least 50% of the total volume of the implant. The implant can comprise, for example, a loop fabric, a knitted fabric, a woven fabric, a braid and/or a felt.

Glycolide/lactide copolymers, preferably polyglactin 910, are suitable, for example, as the material for the predegraded absorbable yarn. Polyglactin 910 is a copolymer of glycolide and lactide in a ratio of 9:1, which is marketed by the Applicant under the name "Vicryl". Polyglactin 910 is degraded by hydrolysis. The degradation products formed, glycolic acid and lactic acid, are metabolized in the body. The absorption time of non-predegraded polyglactin 910 in the body is about 70 days. An absorption time of about 35 days can be achieved, for example, by the treatment for predegradation.

Another example of a material which is suitable as the predegraded absorbable yarn is poly-p-dioxanone. However, the use of absorbable yarns of other materials or the simultaneous use of different yarns or of yarns of different materials in the same implant is conceivable.

The implant is reinforced and/or kept in shape by at least one non-predegraded, preferably absorbable yarn. The stability of the implant can be increased in this manner. Since most of the material of the implant is made of predegraded absorbable yarn, the non-predegraded yarn does not impede or only slightly impedes bone growth, even if it is not absorbable. Poly-p-dioxanone, for example, is suitable for use as non-predegraded yarn.

The advantages of the implant according to the invention can thus be summarized as follows: The implant can be produced in a flat form or in a form which already matches the anatomical circumstances, using an absorbable yarn or various absorbable yarns, the yarns being pretreated such that they still have a sufficiently high mechanical strength, but are absorbed sufficiently rapidly, so that e.g. construction of bone through a defect is not impeded. Typical forms for the implant are, for example, a cylindrical tampon or a disc-shaped tampon. Reliable stopping of seeping haemorrhages from the bone can be achieved with the aid of the implant. The mechanical stability of the implant is sufficiently high for several days, so that effective haemostasis is achieved even in the case of problem haemorrhages. The implant has a high porosity, which allows the penetration of liquid and cellular constituents of blood. The compartmenting of the implant, which is linked to the porosity, stabilizes the blood clot formed.

In the process for the production of an implant according to the invention, at least one absorbable yarn is predegraded by irradiation and/or chemical treatment, and the implant is shaped after said predegradation treatment into an areal or a voluminous form. The predegradation can be carried out in various ways, combinations of various methods, which add to or supplement each other in their action, also being conceivable.

One possibility comprises carrying out the predegradation by irradiation with gamma rays, preferably at an irradiation dose in the range from 10 kGy to 500 kGy. Cobalt irradiation with an irradiation dose of 25 kGy to 100 kGy is particularly suitable.

On one group of polymers, for example on poly-p-dioxanone, the predegradation can also be carried out by irradiation with ultraviolet light, and irradiation with visible light (e.g. with white neon light) also shows an action.

Another possibility is to carry out the predegradation by steeping the absorbable yarn to be predegraded in a hydrolysis buffer. The hydrolysis buffer preferably has a pH in the range from 4 to 10, and the steeping is carried out for a period of time in the range from 10 hours to 300 hours at a temperature in the range from 30°C to 65°C. For this purpose, the absorbable yarn to be predegraded must be hydrolysable, which is the case, for example, for polyglactin 910 and poly-p-dioxanone. A slightly alkaline hydrolysis buffer of about 50°C, in which the absorbable yarn to be predegraded is steeped for about 40 hours to 80 hours, has proved to be particularly suitable, for example, for polyglactin 910. A longer treatment period (for example up to 300 hours) is more suitable for yarns of poly-p-dioxanone.

The process parameters during predegradation can be determined on the basis of test series such that a desired absorption time can be achieved in the case of a particular material for the absorbable yarn. It is particularly advantageous if the strength of the predegraded absorbable yarn is still so high that final production of the implant by textile processing steps, such as looping, knitting, braiding, weaving or also other textile processing steps, is still possible with this yarn. However, the degree of degradation should already be so high that the resulting implants are absorbed significantly faster than conventional implants which comprise no predegraded absorbable yarn.

In order to increase the stability of the implant, in a preferred embodiment at least one predegraded yarn and at least one non-predegraded yarn are knitted, looped, woven or braided together. It is also conceivable that the form of the implant is substantially determined by yarns attached in another controlled manner or, for example, threads produced from such yarns, these yarns being non-predegraded and therefore having a higher strength.

For example, a non-predegraded yarn of poly-p-dioxanone can be incorporated into the implant, the implant being brought into its final form by shrinking the yarn of poly-p-dioxanone at a temperature between 70°C and 110°C. In this case, the properties of yarn of poly-p-dioxanone are utilized for shaping the implant, since untreated (i.e. non-predegraded) yarn of poly-p-dioxanone shows a high shrinkage capacity at temperatures above 70°C and below the melting point (approx. 110°C). Thus, for example, a more rigid implant can be produced from a looser knitted fabric by shrinking the poly-p-dioxanone yarn.

A change in the surface structure is caused in the absorbable yarn by the predegradation process. For example, under high magnification, transversely running gaps and cracks of different degrees can be detected on the surface of individual filaments of polyglactin 910. This change in structure leads to a significantly better and faster stabilization of blood clots. This is possibly due to a better adhesion of platelets at the filaments of the yarns, which has the effect of faster coagulation of the blood.

The invention is described in more detail below with the aid of examples. Embodiment examples for processes for the production of an implant according to the invention and examples which show the influence of process parameters during predegradation on the properties of the implant serve this purpose. Generally, it is to be noted that an implant according to the invention can be constructed in many ways and there is a large number of possibilities for the process step for the predegradation. In an individual case, the absorption time for a given material can be determined experimentally. How this can be done is also explained with the aid of the following examples.

### Example 1

Another possibility of carrying out the predegradation of absorbable yarns comprises irradiation with light, and in particular with ultraviolet light, but also with visible light.

This is investigated in the following by the example of poly-p-dioxanone.

Poly-p-dioxanone, an aliphatic polyester, degrades rapidly by the action of light. If a dye is admixed to the colourless polymer, the rate of degradation by light is reduced. The rate of degradation depends on the intensity and the nature of the light source. The degradation leads to a reduction in the tear strength, and at the same time to a reduction in the molecular weight, which results in accelerated absorption in the body of an implant produced from poly-p-dioxanone. The inherent viscosity (IV) is a measure of the molecular weight; the smaller the IV, the lower the molecular weight of the polymer and the shorter the absorption time.

To investigate the influence of an irradiation with ultraviolet light on poly-p-dioxanone, undyed yarn of poly-p-dioxanone was plied (5 yarns of 60 den each), wound on a frame and exposed to ultraviolet light from a UV light source ("Ultravitalux" lamp from Osram of 100 W output) positioned at a distance of approx. 60 cm.

Table 1 shows the influence of the exposure time on the residual tear strength (related to non-exposed yarn) and on the inherent viscosity.

**Table 1 Influence of an irradiation with UV light on the tear strength and the inherent viscosity (IV) of yarn of undyed poly-p-dioxanone**

| Exposure time [hours] | Tear strength [%] | IV [dl/g] |
|---|---|---|
| 0 | 100 | 1.74 |
| 8 | 94 | 1.41 |
| 24 | 47 | |
| 72 | 12 | 0.57 |

Violet yarn of poly-p-dioxanone which had been coloured with the violet dye "D + C violet 2" (1-hydroxy-4-p-toluidino-anthraquinone), was plied, wound onto a frame and irradiated with an "Ultravitalux" lamp in an analogous manner.

As Table 2 shows, the residual tear strength and the inherent viscosity are significantly higher than for undyed yarn.

**Table 2 Influence of an irradiation with UV light on the tear strength and the inherent viscosity (IV) of yarn of violet poly-p-dioxanone**

| Exposure time [hours] | Tear strength [%] | IV [dl/g] |
|---|---|---|
| 0 | 100 | 1.74 |
| 72 | 88 | 1.54 |
| 120 | 79 | |
| 240 | 46 | 1.44 |

In another experiment, undyed yarn (60 den) was plied, wound onto a frame and exposed to a white neon light source (60 W) at a distance of approx. 60 cm. Table 6 shows the results for the residual tear strength and the inherent viscosity.

**Table 3 Influence of an irradiation with white neon light on the tear strength and the inherent viscosity (IV) of yarn of undyed poly-p-dioxanone**

| Exposure time [hours] | Tear strength [%] | IV [dl/g] |
|---|---|---|
| 0 | 100 | 1.74 |
| 8 | 98 | 1.56 |
| 24 | 95 | |
| 72 | 77 | 1.38 |
| 120 | 43 | 1.14 |

For a given exposure time, the values are between those for undyed yarn and for violet yarn when irradiated with ultraviolet light.

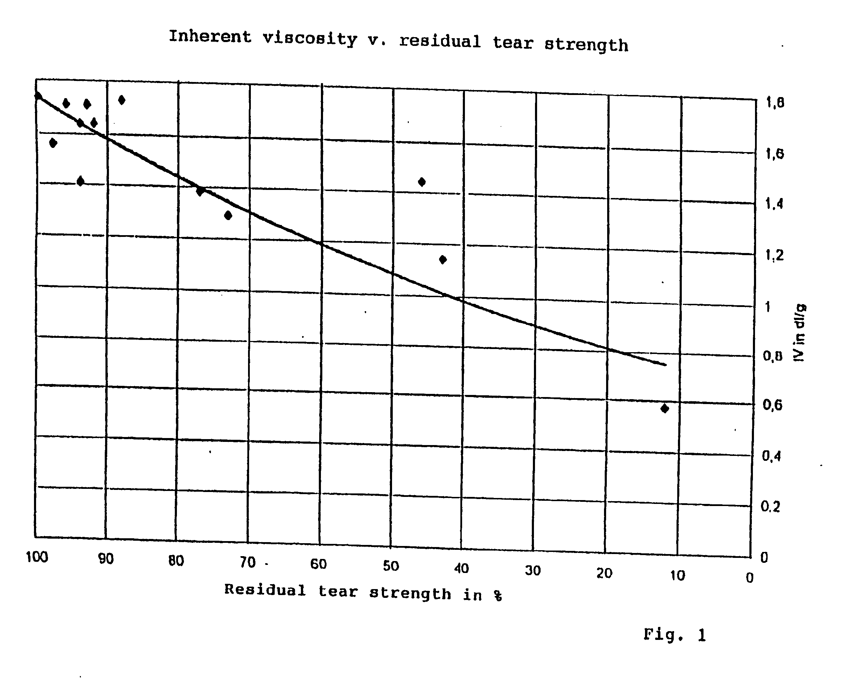

The measured values of the inherent viscosity are plotted against the residual tear strength in Figure 1. The curve drawn through was fitted to the measured data in accordance with an exponential relationship.

The values for the inherent viscosity allow conclusions to be drawn regarding the absorption time of implants in vivo. Various internal studies have shown that yarns of poly-p-dioxanone reach a value for the inherent viscosity of about 0.5 dl/g after an in vivo implantation time of approx. 100 days. From this, it can be concluded that an implant of poly-p-dioxanone, the inherent viscosity of which is already approx. 0.5 dl/g before the implantation, shows an absorption time reduced by approx. 100 days compared with an implant produced from non-predegraded yarn of poly-p-dioxanone. Instead of the absorption time of approx. 180 days customary for such an implant, the absorption time for the implant of predegraded yarn is thus only approx. 80 days.

## Claims

1. Process for the production of an implant,
- wherein at least one absorbable yarn is predegraded by irradiation and/or chemical treatment, and is shaped into an areal or a voluminous form after said predegradation treatment has been performed,
- wherein the implant is reinforced and/or kept in shape by at least one non-predegraded, preferably absorbable yarn,
- the proportion of the predegraded absorbable yarn being at least 50%, based on the total weight of the implant.

2. Process according to claim 1, **characterized in that** the predegradation is carried out by irradiation with gamma rays, preferably at an irradiation dose in the range from 10 kGy to 500 kGy.

3. Process according to claim 1, **characterized in that** the predegradation is carried out by irradiation with ultraviolet light.

4. Process according to one of claims 1 to 3, **characterized in that** the predegradation is carried out by steeping the absorbable yarn to be predegraded in a hydrolysis buffer.

5. Process according to claim 4, **characterized in that** the hydrolysis buffer has a pH in the range from 4 to 10, and **in that** the steeping is carried out for a period of time in the range from 10 hours to 300 hours at a temperature in the range from 30°C to 65°C.

6. Process according to one of claims 1 to 5, **characterized in that** at least one predegraded yarn and at least one non-predegraded yarn are knitted, looped, woven or braided together.

7. Process according to one of claims 1 to 6, **characterized in that** a poly-p-dioxanone yarn, which is preferably non-predegraded, is incorporated into the implant, and **in that** the implant is brought into its final form by shrinking the poly-p-dioxanone yarn at a temperature between 70°C and 110°C.

8. Implant, as manufactured by the process according to one of claims 1 to 7.

9. Implant according to claim 8, **characterized in that** the pore content in the implant is at least 50% of the total volume of the implant.

10. Implant according to claim 8 or 9, **characterized in that** the implant comprises a loop fabric and/or a knitted fabric.

11. Implant according to one of claims 8 or 10, **characterized in that** the implant comprises a woven fabric and/or a braid.

12. Implant according to one of claims 8 to 11, **characterized in that** the implant comprises a felt.

13. Implant according to one of claims 8 to 12, **characterized in that** the implant comprises, as a predegraded absorbable yarn, a predegraded yarn of a glycolide/lactide copolymer, preferably of polyglactin 910.

14. Implant according to one of claims 8 to 13, **characterized in that** the implant comprises, as a predegraded absorbable yarn, a predegraded yarn of poly-p-dioxanone.

15. Implant according to one of claims 8 to 14, **characterized in that** the implant comprises, as non-predegraded yarn, a yarn of poly-p-dioxanone.

## Patentansprüche

1. Verfahren zum Herstellen eines Implantats,
- wobei mindestes ein resorbierbares Garn durch Bestrahlen und/oder chemisches Behandeln vorabgebaut wird und nach Durchführung der Vorabbaubehandlung zu einer flächigen oder volumigen Form gestaltet wird,
- wobei das Implantat mit mindestens einem nicht vorabgebauten, vorzugsweise resorbierbaren Garn verstärkt und/oder in Form gehalten wird,
- wobei der Anteil des vorabgebauten resorbierbaren Garns mindestens 50%, bezogen auf das Gesamtgewicht des Implantats, beträgt.

2. Verfahren nach Anspruch 1, **dadurch gekennzeichnet, dass** das Vorabbauen durch Bestrahlen mit Gammastrahlen durchgeführt wird, vorzugsweise bei einer Bestrahlungsdosis im Bereich von 10 kGy bis 500 kGy.

3. Verfahren nach Anspruch 1, **dadurch gekennzeichnet, dass** das Vorabbauen durch Bestrahlen mit Ultraviolettlicht durchgeführt wird.

4. Verfahren nach einem der Ansprüche 1 bis 3, **dadurch gekennzeichnet, dass** das Vorabbauen durch Einlegen des vorabzubauenden resorbierbaren Garns in einen Hyelrolysepuffer durchgeführt wird.

5. Verfahren nach Anspruch 4, **dadurch gekennzeichnet, dass** der Hydrolysepuffer einen pH-Wert im Bereich von 4 bis 10 hat und dass das Einlegen für eine Zeitdauer im Bereich von 10 Stunden bis 300 Stunden bei einer Temperatur im Bereich von 30°C bis 65°C erfolgt.

6. Verfahren nach einem der Ansprüche 1 bis 5, **dadurch gekennzeichnet, dass** mindestens ein vorabgebautes Garn und mindestens ein nicht vorabgebautes Garn gemeinsam verstrickt, gewirkt, verwebt oder verflochten werden.

7. Verfahren nach einem der Ansprüche 1 bis 6, **dadurch gekennzeichnet, dass** in das Implantat ein vorzugsweise nicht vorabgebautes Garn aus Poly-p-dioxanon eingearbeitet wird und dass das Implantat in seine endgültige Form gebracht wird, indem das Garn aus Poly-p-dioxanon bei einer Temperatur zwischen 70°C und 110°C geschrumpft wird.

8. Implantat, hergestellt gemäß dem Verfahren nach einem der Ansprüche 1 bis 7.

9. Implantat nach Anspruch 8, **dadurch gekennzeichnet, dass** der Porenanteil in dem Implantat mindestens 50% des Gesamtvolumens des Implantats beträgt.

10. Implantat nach Anspruch 8 oder 9, **dadurch gekennzeichnet, dass** das Implantat ein Gewirke und/oder ein Gestricke aufweist.

11. Implantat nach einem der Ansprüche 8 bis 10, **dadurch gekennzeichnet, dass** das Implantat ein Gewebe und/oder ein Geflecht aufweist.

12. Implantat nach einem der Ansprüche 8 bis 11, **dadurch gekennzeichnet, dass** das Implantat einen Filz aufweist.

13. Implantat nach einem der Ansprüche 8 bis 12, **dadurch gekennzeichnet, dass** das Implantat als vorabgebautes resorbierbares Garn ein vorabgebautes Garn aus einem Glykolid/Lactid-Copolymer, vorzugsweise aus Polyglactin 910, aufweist.

14. Implantat nach einem der Ansprüche 8 bis 13, **dadurch gekennzeichnet, dass** das Implantat als vorabgebautes resorbierbares Garn ein vorabgebautes Garn aus Poly-p-dioxanon aufweist.

15. Implantat nach einem der Ansprüche 8 bis 14, **dadurch gekennzeichnet, dass** das Implantat als nicht vorabgebautes Garn ein Garn aus Poly-p-dioxanon aufweist.

## Revendications

1. Procédé pour la production d'un implant,
- dans lequel au moins un fil résorbable par irradiation et/ou traitement chimique est préalablement dégradé, et est formé en une forme surfacique ou volumique après que ledit traitement de dégradation préalable ait été effectué,
- dans lequel l'implant est renforcé et/ou maintenu en forme par au moins un fil non préalablement dégradé, de préférence résorbable,
- la proportion de fil résorbable préalablement dégradé étant d'au moins 50 %, sur la base du poids total de l'implant.

2. Procédé selon la revendication 1, **caractérisé en ce que** la dégradation préalable est effectuée par irradiation avec des rayons gamma, de préférence à une dose d'irradiation dans la plage de 10 kGy à 500 kGy.

3. Procédé selon la revendication 1, **caractérisé en ce que** la dégradation préalable est effectuée par irradiation avec une lumière ultraviolette.

4. Procédé selon l'une des revendications 1 à 3, **caractérisé en ce que** la dégradation préalable est effectuée par trempage du fil résorbable à dégrader préalablement dans un tampon d'hydrolyse.

5. Procédé selon la revendication 4, **caractérisé en ce que** le tampon d'hydrolyse a un pH dans la plage de 4 à 10, et **en ce que** le trempage est effectué pendant une durée dans la plage de 10 heures à 300 heures à une température dans la plage de 30°C à 65°C.

6. Procédé selon l'une des revendications 1 à 5, **caractérisé en ce qu'**au moins un fil préalablement dégradé et au moins un fil non préalablement dégradé sont tricotés, bouclés, tissés ou tressés ensemble.

7. Procédé selon l'une des revendications 1 à 6, **caractérisé en ce qu'**un fil en poly-p-dioxanone, qui est de préférence non préalablement dégradé, est incorporé dans l'implant, et **en ce que** l'implant est amené à sa forme finale par contraction du fil de poly-p- dioxanone à une température entre 70°C et 110°C.

8. Implant, fabriqué par le procédé selon l'une des revendications 1 à 7.

9. Implant selon la revendication 8, **caractérisé en ce que** la porosité dans l'implant est d'au moins 50 % du volume total de l'implant.

10. Implant selon la revendication 8 ou 9, **caractérisé en ce que** l'implant comprend un tissu bouclé et/ou un tissu tricoté.

11. Implant selon l'une des revendications 8 à 10, **caractérisé en ce que** l'implant comprend un tissu tissé et/ou une tresse.

12. Implant selon l'une des revendications 8 à 11, **caractérisé en ce que** l'implant comprend un feutre.

13. Implant selon l'une des revendications 8 à 12, **caractérisé en ce que** l'implant comprend, en tant que fil résorbable préalablement dégradé, un fil préalablement dégradé d'un copolymère glycolide/lactide, de préférence de polyglactine 910.

14. Implant selon l'une des revendications 8 à 13, **caractérisé en ce que** l'implant comprend, en tant que fil résorbable préalablement dégradé, un fil préalablement dégradé de poly-p-dioxanone.

15. Implant selon l'une des revendications 8 à 14, **caractérisé en ce que** l'implant comprend, en tant que fil non préalablement dégradé, un fil de poly-p-dioxanone.
